# EUROPEAN PATENT APPLICATION

(11) **EP 4 656 717 A1**
(43) Date of publication of application: **03.12.2025**
(21) Application number: 25179655.3
(22) Date of filing: 29.05.2025
(51) Int. Cl.: C12N 9/04

(54) **GLUCOSE OXIDASE MUTANT, METHOD FOR PREPARING, AND USES**

(30) Priority: 31.05.2024 CN 202410707813
(71) Applicant: Shanghai United Imaging Microelectronics Technology Co., Ltd., Shanghai 201899 (CN)
(72) Inventor: JIA, Jie, Shanghai, 201899 (CN)
(74) Representative: Murgitroyd & Company

(57) **Abstract**

The present application provides a glucose oxidase mutant, the glucose oxidase mutant has at least one of the following mutations compared to wild-type glucose oxidase: G108K, G419K; or the glucose oxidase mutant has at least one of the following mutations compared to wild-type glucose oxidase: G108D, G419D; the amino acid sequence of the wild-type glucose oxidase is set forth in SEQ ID NO:1.

## Description

This application claims priority to Chinese Patent Application No. 202410707813.8, filed on May,31 2024.

### REFERENCE TO SEQUENCE LISTING

This application includes a Sequence Listing filed electronically as an XML file name "SP25719871EP", created on May 28, 2025, with a size of 21,399 bytes. The Sequence Listing is incorporated herein by reference.

### TECHNICAL FIELD

The present application relates to biotechnology, and in particular, to a glucose oxidase mutant, a method for preparing, and uses.

### BACKGROUND

Glucose oxidase (GOx) is a well-characterized aerobic dehydrogenase that utilizes molecular oxygen as an electron acceptor under aerobic conditions to catalyze the conversion of β-D-glucose to D-gluconolactone and hydrogen peroxide, the latter of which is subsequently hydrolyzed to gluconic acid and water. In electrochemical sensors, the currently employed glucose oxidases suffer from low electron transfer efficiency, prompting substantial efforts by researchers to enhance such efficiency. Existing immobilization methods for glucose oxidase primarily include adsorption, covalent binding, cross-linking, and embedding. However, owing to the intrinsic structural characteristics of glucose oxidase, these methods still present challenges such as long electron transport pathways and low electron transfer efficiency.

Therefore, it is desirable to provide a glucose oxidase mutant, a method for preparing, and uses.

### SUMMARY

An aspect of the present application may provide:
A glucose oxidase mutant, wherein the glucose oxidase mutant has at least one of the following mutations compared to wild-type glucose oxidase: G108K, G419K; or the glucose oxidase mutant has at least one of the following mutations compared to wild-type glucose oxidase: G108D, G419D; the amino acid sequence of the wild-type glucose oxidase is set forth in SEQ ID NO: 1.

In some embodiments, the amino acid sequence of the glucose oxidase mutant is set forth in SEQ ID NO:2.

In some embodiments, the amino acid sequence of the glucose oxidase mutant is set forth in SEQ ID NO:3.

Another aspect of the present application may provide a nucleic acid encoding the glucose oxidase mutant as mentioned before.

Another aspect of the present application may provide a vector comprising the nucleic acid as mentioned before.

Another aspect of the present application may provide a host cell comprising a vector as mentioned before.

In some embodiments, the host cell is a recipient cell.

In some embodiments, the recipient cell is selected from the group comprising: *Escherichia coli, Agrobacterium, Saccharomyces cerevisiae, Pichia pastoris, Aspergillus niger*, an animal cell, or a plant cell.

In some embodiments, the recipient cell is selected from the group comprising: *Escherichia coli* DH5α, *Escherichia coli* Top10, *Escherichia coli* Origami(DE3), *Agrobacterium* AGL1, *Aspergillus niger*, *Pichia pastoris* GS115, or *Pichia pastoris* SMD1168.

Another aspect of the present application may provide a method for preparing a glucose oxidase mutant, the method comprising introducing at least one of the following mutations: G108K, G419K into a glucose oxidase having an amino acid sequence as set forth in SEQ ID NO:1; or introducing at least one of the following mutations: G108D, G419D into the glucose oxidase.

In some embodiments, the method comprising steps S100 to S300:
step S100: Preparing a linear recombinant vector, the linear recombinant vector comprising the glucose oxidase gene;
step S200: PCR amplifying the linear recombinant vector using primers as set forth in SEQ ID NOs: 10-13 and SEQ ID NOs: 14-17 to obtain a first mutant recombinant vector and a second mutant recombinant vector, respectively.
step S300: Transforming the first mutant recombinant vector and the second mutant recombinant vector into host cells, culturing the host cells, and preparing the glucose oxidase mutant.
In some embodiments, each 20µL reaction system comprises 1 to 5µL of the glucose oxidase target fragment.

In some embodiments, each 20µL reaction system comprises 1 to 5µL of a linearized connecting carrier.

In some embodiments, the PCR amplification reaction is programmed as: pre-denaturation at 95°C for 3 to 5 minutes; denaturation at 95°C for 10 to 30 seconds, annealing at 56°C to 60°C for 10 to 30 seconds, extension at 72°C for 1 to 5 minutes, for a total of 30-35 cycles; and finally, extension at 72°C for 3 to 7 minutes, and storing the PCR amplification products at 4°C.

Another aspect of the present application may provide use of the glucose oxidase mutant as mentioned before in the preparation of a biosensing element, wherein the biosensing element comprises a biological enzyme electrode or a biosensor.

Another aspect of the present application may provide a biological enzyme electrode, comprising a base electrode loaded with a modified material for modifying the electrode;

the modified material comprises at least one of the glucose oxidase mutant as mentioned before.

Another aspect of the present application may provide a method for preparing the biological enzyme electrode as mentioned before, the method comprising co-incubating a substrate electrode with the glucose oxidase mutant as mentioned before.

In some embodiments, the method comprising coating a binder on the substrate electrode.

In some embodiments, the binder comprises at least one of metal ions, proteins, and small molecule substances.

Another aspect of the present application may provide a biosensor comprising the biological enzyme electrode as mentioned before.

Additional features will be set forth in part in the description which follows, and in part will become apparent to those skilled in the art upon examination of the following and the accompanying drawings or may be learned by production or operation of the examples. The features of the present application may be realized and attained by practice or use of various aspects of the methodologies, instrumentalities, and combinations set forth in the detailed examples discussed below.

### BRIEF DESCRIPTION OF THE DRAWINGS

The present application is further illustrated in terms of exemplary embodiments. These exemplary embodiments are described in detail with reference to according to the drawings. These embodiments are non-limiting exemplary embodiments, in which like reference numerals represent similar structures, and wherein:

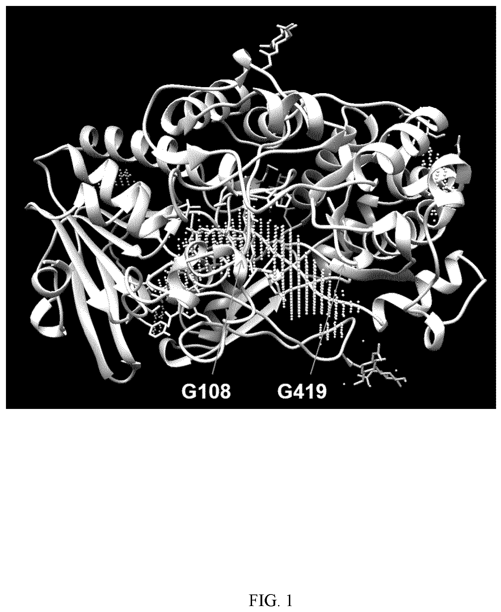

FIG. 1 depicts the positions of glycine residues at amino acid positions 108 and 419 in the three-dimensional structure of the wild-type glucose oxidase described in Example 1.

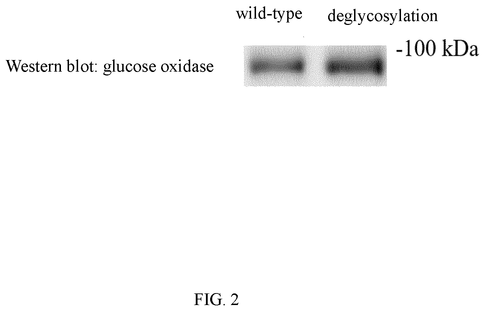

FIG. 2 illustrates the SDS-PAGE electrophoresis pattern of the wild-type and mutant glucose oxidase from Example 1.

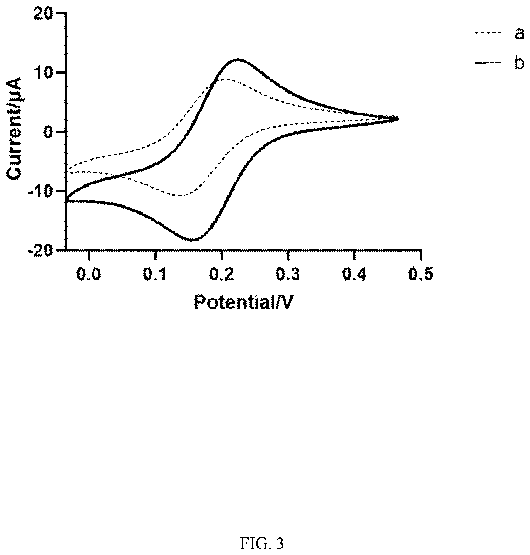

FIG. 3 presents the redox potential detection results for the wild-type and mutant glucose oxidase of Example 1, where (a) corresponds to the wild-type glucose oxidase and (b) corresponds to the mutant glucose oxidase, with the horizontal axis representing voltage and the vertical axis representing current.

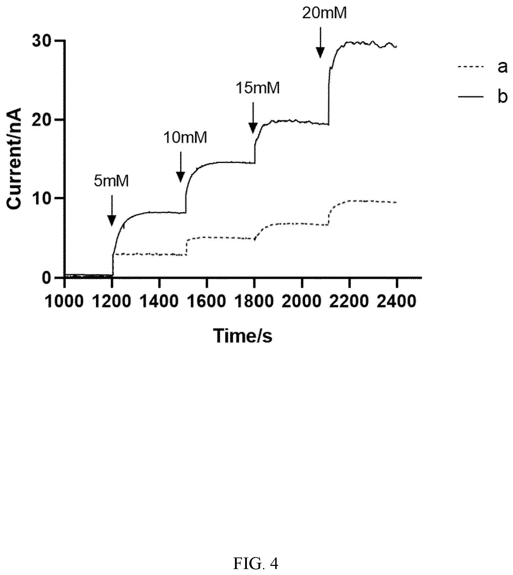

FIG. 4 shows the relationship of current to glucose concentration for the wild-type and mutant glucose oxidase on the bioelectrochemical sensor of Example 1, where (a) represents the wild-type glucose oxidase and (b) represents the mutant glucose oxidase, with the horizontal axis denoting time and the vertical axis denoting current.

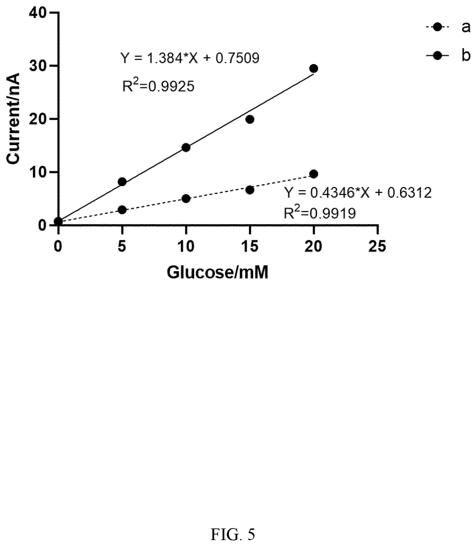

FIG. 5 displays the linear correlation plot of current versus glucose concentration for the wild-type and mutant glucose oxidase on the bioelectrochemical sensor of Example 1, where (a) corresponds to the wild-type glucose oxidase and (b) corresponds to the mutant glucose oxidase, with the horizontal axis indicating concentration and the vertical axis indicating current.

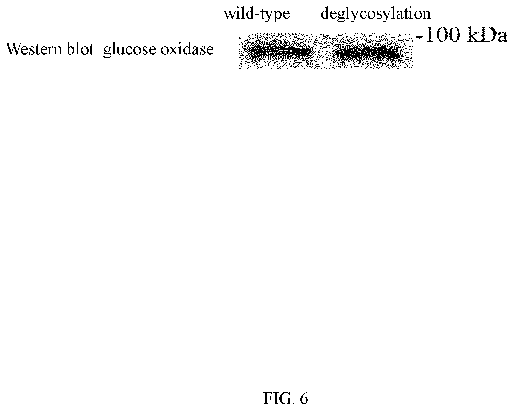

FIG. 6 shows the SDS-PAGE electrophoresis pattern of the wild-type and mutant glucose oxidase in Example 2.

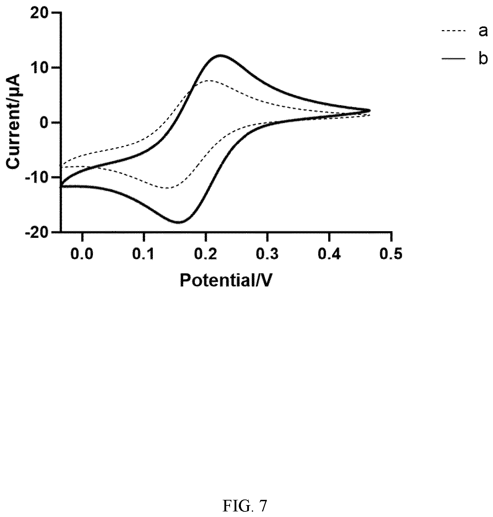

FIG.7 presents the redox potential detection results for the wild-type and mutant glucose oxidase in Comparative Example 2, where (a) represents the wild-type glucose oxidase and (b) represents the mutant glucose oxidase, with the horizontal axis representing voltage and the vertical axis representing current.

### DETAILED DESCRIPTION

In the following detailed description, numerous specific details are set forth by way of examples in order to provide a thorough understanding of the relevant disclosure. However, it should be apparent to those skilled in the art that the present application may be practiced without such details. In other instances, well-known methods, procedures, systems, components, and/or circuitry have been described at a relatively high level, without detail, in order to avoid unnecessarily obscuring aspects of the present application. Various modifications to the disclosed embodiments will be readily apparent to those skilled in the art, and the general principles defined herein may be applied to other embodiments and applications without departing from the spirit and scope of the present application. Thus, the present application is not limited to the embodiments shown, but is to be accorded the widest scope consistent with the claims.

The terminology used herein is for the purpose of describing particular example embodiments only and is not intended to be limiting. As used herein, the singular forms "a", "an", and "the" may be intended to include the plural forms as well, unless the context clearly indicates otherwise. It will be further understood that the terms "comprise", "comprises", and/or "comprising", "include" ,"includes", and/or "including", when used in this specification, specify the presence of stated features, integers, steps, operations, elements, and/or components, but do not preclude the presence or addition of one or more other features, integers, steps, operations, elements, components, and/or groups thereof.

It will be understood that the terms "system", "engine", "unit", "module", and/or "block" used herein are one method to distinguish different components, elements, parts, sections or assemblies of different levels in ascending order. However, the terms may be displaced by another expression if they achieve the same purpose.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items. The terms "pixel" and "voxel" in the present application are used interchangeably to refer to an element of an image.

These and other features, and characteristics of the present application, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present application. It is understood that the drawings are not to scale.

Biological enzymes are defined as biological macromolecules exhibiting catalytic activity, including proteins and/or nucleic acids, and generally encompass redox enzymes, transferases, hydrolases, lyases, isomerases, and/or synthases.

Glucose oxidase (GOx) is a well-characterized aerobic dehydrogenase that utilizes molecular oxygen as an electron acceptor under aerobic conditions to catalyze the conversion of β-D-glucose to D-gluconolactone and hydrogen peroxide, the latter of which is subsequently hydrolyzed to gluconic acid and water.

A biosensor is an important detection tool in which biological materials such as microorganisms, nucleic acids, proteins, enzymes, and organelles are immobilized on the surface of recognition elements in a specific manner, for specifically recognizing a substrate and accompanied by the occurrence of a physical or chemical change, during which a biological activity signal is converted into a monitorable electrical or spectral signal. During the reaction, an electron mediator is involved, and the electrochemical detection system captures changes in the electron mediator, generating currents of varying intensities that depend on the substrate concentration.

Due to the low electron mediator efficiency of currently used glucose oxidase in electrochemical sensors, researchers have undertaken substantial efforts to enhance such efficiency. Existing immobilization methods for glucose oxidase include primarily adsorption, covalent binding, cross-linking, and embedding. However, owing to the intrinsic structural characteristics of glucose oxidase, these methods still present challenges such as elongated electron mediator pathways and diminished electron mediator efficiency.

In currently employed second-generation electrochemical sensors, even when electron mediators are added, the inconsistent structural orientations of biological enzymes following immobilization on the sensor-based on protein structure analysis-result in the electron mediator primarily transferring from the substrate-binding pocket of the biological enzymes to the sensor matrix. This structural inconsistency leads to an elongated electron mediator path and diminished electron mediator efficiency.

The applicant has engineered the glucose oxidase via protein engineering, resulting in the immobilized glucose oxidase having a uniform structural orientation and minimal activity loss. The glucose oxidase mutant exhibits a shorter electron mediator distance and improved electron mediator efficiency, which is benefit to its use in biosensors.

An embodiment of the present application provides a method for preparing a biological enzyme mutant, comprising steps A and B.
step A: Identify the substrate-binding pocket of the biological enzyme; step B: Mutate the substrate-binding pocket of the biological enzyme identified in Step A.

Specifically, in Step A, substrate-binding pocket or substrate-binding pocket site is obtained or predicted based on: (1) protein three-dimensional structure; (2) protein gene sequence; and/or (3) physicochemical properties of amino acid residues.

In some embodiments, the substrate-binding pocket sites include hydrophobic amino acid sequences.

It is understood that the substrate typically interacts with amino acid residues via hydrogen bonds, ionic bonds, van der Waals forces, and hydrophobic interactions.

In some embodiments, the hydrophobic amino acid sequence comprises at least one of tryptophan, phenylalanine, valine, leucine, isoleucine, alanine, proline, or methionine.

In some embodiments, the amino acid sequence involved in hydrogen bond binding comprises at least one of serine, threonine, aspartic acid, glutamic acid, lysine, tyrosine, or tryptophan.

In some embodiments, the mutation methods include at least one of site-directed mutagenesis and homologous recombination.

Specifically, the mutations involve altering amino acids on the exterior of the binding pocket to at least one of alanine, isoleucine, leucine, valine, methionine, phenylalanine, tryptophan, tyrosine, cysteine, glutamine, arginine, histidine, lysine, aspartic acid, glutamic acid, glycine, or proline.

In some embodiments, step B further includes a step of adding a specific tag to the mutated substrate-binding pocket of the biological enzyme.

In some embodiments, the specific tags comprise at least one of His tags, GST tags, Flag tags, Strep tags, MBP tags, CBP tags, CBD tags, or Halo tags.

Specifically, the steps for adding the specific tag include covalently attaching the tag to the amino acid residue via a condensation reaction.

In some embodiments, the condensing agent includes carbodiimide-type and/or onium salt-type compounds.

With reference to FIG. 1, an embodiment of the present application provides a glucose oxidase mutant having at least one of the following mutations compared to the wild-type glucose oxidase: G108K, G419K; or the glucose oxidase mutant having at least one of the following mutations compared to the wild-type glucose oxidase: G108D, G419D.

The amino acid sequence of the aforementioned wild-type glucose oxidase is set forth in SEQ ID NO: 1.

Specifically, the amino acid sequence set forth in SEQ ID NO:1 is:

MQTLLVSSLVVSLAAALPHYIRSNGIEASLLTDPKDVSGRTVDYIIAGGGLTGLTTA
ARLTENPNISVLVIESGSYESDRGPIIEDLNAYGDIFGSSVDHAYETVELATNNQTA
LIRSGNGLGGSTLVNGGTWTRPHKAQVDSWETVFGNEGWNWDNVAAYSLQAER
ARAPNAKQIAAGHYFNASCHGVNGTVHAGPRDTGDDYSPIVKALMSAVEDRGVP
TKKDFGCGDPHGVSMFPNTLHEDQVRSDAAREWLLPNYQRPNLQVLTGQYVGK
VLLSQNGTTPRAVGVEFGTHKGNTHNVYAKHEVLLAAGSAVSPTILEYSGIGMKSI
LEPLGIDTVVDLPVGLNLQDQTTATVRSRITSAGAGQGQAAWFATFNETFGDYSE
KAHELLNTKLEQWAEEAVARGGFHNTTALLIQYENYRDWIVNHNVAYSELFLDT
AGVASFDVWDLLPFTRGYVHILDKDPYLHHFAYDPQYFLNELDLLGQAAATQLA
RNISNSGAMQTYFAGETIPGDNLAYDADLSAWTEYIPYHFRPNYHGVGTCSMMPK
EMGGVVDNAARVYGVQGLRVIDGSIPPTQMSSHVMTVFYAMALKISDAILEDYAS
MQ.

In some embodiments, based on the three-dimensional structure of glucose oxidase GOX (amino acid sequence set forth in SEQ ID NO: 1), glycines at positions 108 and 419 of the substrate-binding pocket were selected for mutation.

In some embodiments, the glucose oxidase mutant has the following mutations compared to the wild-type glucose oxidase: G108K and G419K, resulting in a mutant with an amino acid sequence set forth in SEQ ID NO:2.

Specifically, the amino acid sequence set forth in SEQ ID NO:2 is:

MQTLLVSSLVVSLAAALPHYIRSNGIEASLLTDPKDVSGRTVDYIIAGGGLTGLTTA
ARLTENPNISVLVIESGSYESDRGPIIEDLNAYGDIFGSSVDHAYETVELATNNQTA
LIRSGNGLGGSTLVNKGTWTRPHKAQVDSWETVFGNEGWNWDNVAAYSLQAER
ARAPNAKQIAAGHYFNASCHGVNGTVHAGPRDTGDDYSPIVKALMSAVEDRGVP
TKKDFGCGDPHGVSMFPNTLHEDQVRSDAAREWLLPNYQRPNLQVLTGQYVGK
VLLSQNGTTPRAVGVEFGTHKGNTHNVYAKHEVLLAAGSAVSPTILEYSGIGMKSI
LEPLGIDTVVDLPVGLNLQDQTTATVRSRITSAGAGQGQAAWFATFNETFGDYSE
KAHELLNTKLEQWAEEAVARGGFHNTTALLIQYENYRDWIVNHNVAYSELFLDT
AKVASFDVWDLLPFTRGYVHILDKDPYLHHFAYDPQYFLNELDLLGQAAATQLA
RNISNSGAMQTYFAGETIPGDNLAYDADLSAWTEYIPYHFRPNYHGVGTCSMMPK
EMGGVVDNAARVYGVQGLRVIDGSIPPTQMSSHVMTVFYAMALKISDAILEDYAS
MQ.

In some embodiments, the glucose oxidase mutant has the following mutations compared to the wild-type glucose oxidase: G108D and G419D, resulting in a mutant with an amino acid sequence set forth in SEQ ID NO:3.

Specifically, the amino acid sequence set forth in SEQ ID NO:3 is:

MQTLLVSSLVVSLAAALPHYIRSNGIEASLLTDPKDVSGRTVDYIIAGGGLTGLTTA
ARLTENPNISVLVIESGSYESDRGPIIEDLNAYGDIFGSSVDHAYETVELATNNQTA
LIRSGNGLGGSTLVNDGTWTRPHKAQVDSWETVFGNEGWNWDNVAAYSLQAER
ARAPNAKQIAAGHYFNASCHGVNGTVHAGPRDTGDDYSPIVKALMSAVEDRGVP
TKKDFGCGDPHGVSMFPNTLHEDQVRSDAAREWLLPNYQRPNLQVLTGQYVGK
VLLSQNGTTPRAVGVEFGTHKGNTHNVYAKHEVLLAAGSAVSPTILEYSGIGMKSI
LEPLGIDTVVDLPVGLNLQDQTTATVRSRITSAGAGQGQAAWFATFNETFGDYSE
KAHELLNTKLEQWAEEAVARGGFHNTTALLIQYENYRDWIVNHNVAYSELFLDT
ADVASFDVWDLLPFTRGYVHILDKDPYLHHFAYDPQYFLNELDLLGQAAATQLA
RNISNSGAMQTYFAGETIPGDNLAYDADLSAWTEYIPYHFRPNYHGVGTCSMMPK
EMGGVVDNAARVYGVQGLRVIDGSIPPTQMSSHVMTVFYAMALKISDAILEDYAS
MQ.

A first embodiment of the present application further provides a nucleic acid fragment encoding for mediating the secretion and expression of the aforementioned glucose oxidase by a glucose oxidase mutant in a host cell.

In some embodiments, the nucleic acid comprises a nucleic acid fragment encoding a glucose oxidase mutant with an amino acid sequence such as that set forth in SEQ ID NO:2.

Optionally, the nucleotide sequence of the nucleic acid encoding the amino acid sequence of the glucose oxidase mutant set forth in SEQ ID NO:2 is set forth in SEQ ID NO:4. It is understood that, due to the degeneracy of the codons, in other embodiments, the nucleotide sequence of the nucleic acid encoding the amino acid sequence of the glucose oxidase mutant set forth in SEQ ID NO:2 is not limited to the above and can be other sequences.

Specifically, the above nucleotide sequence set forth in SEQ ID NO:4 is: 5'-

ATGCAGACTCTCCTTGTGAGCTCGCTTGTGGTCTCCCTCGCTGCGGCCCTGCCA
CACTACATCAGGAGCAATGGCATTGAAGCCAGCCTCCTGACTGATCCCAAGGA
TGTCTCCGGCCGCACGGTCGACTACATCATCGCTGGTGGAGGTCTGACTGGAC
TCACCACCGCTGCTCGTCTGACGGAGAACCCCAACATCAGTGTGCTCGTCATC
GAAAGTGGCTCCTACGAGTCGGACAGAGGTCCTATCATTGAGGACCTGAACGC
CTACGGCGACATCTTTGGCAGCAGTGTAGACCACGCCTACGAGACCGTGGAGC
TCGCTACCAACAATCAAACCGCGCTGATCCGCTCCGGAAATGGTCTCGGTGGC
TCTACTCTAGTGAATAAAGGCACCTGGACTCGCCCCCACAAGGCACAGGTTGA
CTCTTGGGAGACTGTCTTTGGAAATGAGGGCTGGAACTGGGACAATGTGGCCG
CCTACTCCCTCCAGGCTGAGCGTGCTCGCGCACCAAATGCCAAACAGATCGCT
GCTGGCCACTACTTCAACGCATCCTGCCATGGTGTTAATGGTACTGTCCATGCC
GGACCCCGCGACACCGGCGATGACTATTCTCCCATCGTCAAGGCTCTCATGAG
CGCTGTCGAAGACCGGGGCGTTCCCACCAAGAAAGACTTCGGATGCGGTGACC
CCCATGGTGTGTCCATGTTCCCCAACACCTTGCACGAAGACCAATATGTTGGTA
AGGTGCTCCTTAGCCAGAACGGCACCACCCCTCGTGCCGTTGGCGTGGAATTC
GGCACCCACAAGGGCAACACCCACAACGTTTACGCTAAGCACGAGGTCCTCCT
GGCCGCGGGCTCCGCTGTCTCTCCCACAATCCTCGAATATTCCGGTATCGGAAT
GAAGTCCATCCTGGAGCCCCTTGGTATCGACACCGTCGTTGACCTGCCCGTCGG
CTTGAACCTGCAGGACCAGACCACCGCTACCGTCCGCTCCCGCATCACCTCTGC
TGGTGCAGGACAGGGACAGGCCGCTTGGTTCGCCACCTTCAACGAGACCTTTG
GTGACTATTCCGAAAAGGCACACGAGCTGCTCAACACCAAGCTGGAGCAGTGG
GCCGAAGAGGCCGTCGCCCGTGGCGGATTCCACAACACCACCGCCTTGCTCAT
CCAGTACGAGAACTACCGCGACTGGATTGTCAACCACAACGTCGCGTACTCGG
AACTCTTCCTCGACACTGCCAAAGTAGCCAGCTTCGATGTGTGGGACCTTCTGC

CCTTCACCCGAGGATACGTTCACATCCTCGACAAGGACCCCTACCTTCACCACT
TCGCCTACGACCCTCAGTACTTCCTCAACGAGCTGGACCTGCTCGGTCAGGCTG
CCGCTACTCAACTGGCCCGCAACATCTCCAACTCCGGTGCCATGCAGACCTACT
TCGCTGGGGAGACTATCCCCGGTGATAACCTCGCGTATGATGCCGATTTGAGC
GCCTGGACTGAGTACATCCCGTACCACTTCCGTCCTAACTACCATGGCGTGGGT
ACTTGCTCCATGATGCCGAAGGAGATGGGCGGTGTTGTTGATAATGCTGCCCG
TGTGTATGGTGTGCAGGGACTGCGTGTCATTGATGGTTCTATTCCTCCTACGCA
AATGTCGTCCCATGTCATGACGGTGTTCTATGCCATGGCGCTAAAAATTTCGGA
TGCTATCTTGGAAGATTATGCTTCCATGCAGTGA-3’.

In some embodiments, the nucleic acid includes a fragment of the nucleic acid encoding an amino acid sequence such as that of the glucose oxidase mutant set forth in SEQ ID NO:3.

Optionally, the nucleotide sequence of the nucleic acid encoding the amino acid sequence of the glucose oxidase mutant set forth in SEQ ID NO:3 is set forth in SEQ ID NO:5. It is understood that, due to the degeneracy of the codons, in other embodiments, the nucleotide sequence of the nucleic acid encoding the amino acid sequence of the glucose oxidase mutant set forth in SEQ ID NO:3 is not limited to the above and can be other sequences.

Specifically, the above nucleotide sequence set forth in SEQ ID NO:5 is: 5'-

ATGCAGACTCTCCTTGTGAGCTCGCTTGTGGTCTCCCTCGCTGCGGCCCTGCCA
CACTACATCAGGAGCAATGGCATTGAAGCCAGCCTCCTGACTGATCCCAAGGA
TGTCTCCGGCCGCACGGTCGACTACATCATCGCTGGTGGAGGTCTGACTGGAC
TCACCACCGCTGCTCGTCTGACGGAGAACCCCAACATCAGTGTGCTCGTCATC
GAAAGTGGCTCCTACGAGTCGGACAGAGGTCCTATCATTGAGGACCTGAACGC
CTACGGCGACATCTTTGGCAGCAGTGTAGACCACGCCTACGAGACCGTGGAGC
TCGCTACCAACAATCAAACCGCGCTGATCCGCTCCGGAAATGGTCTCGGTGGC
TCTACTCTAGTGAATGACGGCACCTGGACTCGCCCCCACAAGGCACAGGTTGA
CTCTTGGGAGACTGTCTTTGGAAATGAGGGCTGGAACTGGGACAATGTGGCCG
CCTACTCCCTCCAGGCTGAGCGTGCTCGCGCACCAAATGCCAAACAGATCGCT
GCTGGCCACTACTTCAACGCATCCTGCCATGGTGTTAATGGTACTGTCCATGCC
GGACCCCGCGACACCGGCGATGACTATTCTCCCATCGTCAAGGCTCTCATGAG
CGCTGTCGAAGACCGGGGCGTTCCCACCAAGAAAGACTTCGGATGCGGTGACC
CCCATGGTGTGTCCATGTTCCCCAACACCTTGCACGAAGACCAATATGTTGGTA
AGGTGCTCCTTAGCCAGAACGGCACCACCCCTCGTGCCGTTGGCGTGGAATTC
GGCACCCACAAGGGCAACACCCACAACGTTTACGCTAAGCACGAGGTCCTCCT

GGCCGCGGGCTCCGCTGTCTCTCCCACAATCCTCGAATATTCCGGTATCGGAAT
GAAGTCCATCCTGGAGCCCCTTGGTATCGACACCGTCGTTGACCTGCCCGTCGG
CTTGAACCTGCAGGACCAGACCACCGCTACCGTCCGCTCCCGCATCACCTCTGC
TGGTGCAGGACAGGGACAGGCCGCTTGGTTCGCCACCTTCAACGAGACCTTTG
GTGACTATTCCGAAAAGGCACACGAGCTGCTCAACACCAAGCTGGAGCAGTGG
GCCGAAGAGGCCGTCGCCCGTGGCGGATTCCACAACACCACCGCCTTGCTCAT
CCAGTACGAGAACTACCGCGACTGGATTGTCAACCACAACGTCGCGTACTCGG
AACTCTTCCTCGACACTGCCGACGTAGCCAGCTTCGATGTGTGGGACCTTCTGC
CCTTCACCCGAGGATACGTTCACATCCTCGACAAGGACCCCTACCTTCACCACT
TCGCCTACGACCCTCAGTACTTCCTCAACGAGCTGGACCTGCTCGGTCAGGCTG
CCGCTACTCAACTGGCCCGCAACATCTCCAACTCCGGTGCCATGCAGACCTACT
TCGCTGGGGAGACTATCCCCGGTGATAACCTCGCGTATGATGCCGATTTGAGC
GCCTGGACTGAGTACATCCCGTACCACTTCCGTCCTAACTACCATGGCGTGGGT
ACTTGCTCCATGATGCCGAAGGAGATGGGCGGTGTTGTTGATAATGCTGCCCG
TGTGTATGGTGTGCAGGGACTGCGTGTCATTGATGGTTCTATTCCTCCTACGCA
AATGTCGTCCCATGTCATGACGGTGTTCTATGCCATGGCGCTAAAAATTTCGGA
TGCTATCTTGGAAGATTATGCTTCCATGCAGTGA-3’.

It should be noted that in the above nucleic acids, the nucleic acid fragments encoding the glucose oxidase mutant with the amino acid sequence set forth in SEQ ID NO:2 and the nucleic acid fragments encoding the glucose oxidase mutant with the amino acid sequence set forth in SEQ ID NO:3 may be present in different reagent systems (for example, the above two nucleic acid fragments are located on different expression vectors), or they may be present simultaneously in the same reagent system (for example, the two nucleic acid fragments are on the same expression vector).

**In** some embodiments, the nucleic acid of either of the above embodiments further includes a transcription element, such as a promoter and a terminator. It is understood that in some embodiments, the nucleic acid of any of the above embodiments may not include the transcription element. In such a case, the nucleic acid can be inserted into a vector with the corresponding transcription element for expression when in use.

Furthermore, an embodiment of the present invention provides a vector comprising the nucleic acid of any of the above embodiments, wherein the encoded amino acid sequence is a nucleic acid fragment of a glucose oxidase mutant as set forth in SEQ ID NO:2.

**In** some other embodiments, the vector comprises the nucleic acid of either of the above embodiments, wherein the encoded amino acid sequence is a nucleic acid fragment of the glucose oxidase mutant as set forth in SEQ ID NO:3.

In one embodiment, the vector is the pMJB1 vector. Of course, in other embodiments, the vector is not limited to the pMJB1 vector and can be other vectors. Design site-directed mutagenesis primers, perform site-directed mutagenesis PCR amplification to obtain the target fragment, clone the target sequence of the glucose oxidase mutant into the DPN1 digestion site, transform or transfect the expression vector containing glucose oxidase into the host cell, and express the target protein.

Furthermore, an embodiment of the present invention provides a host cell transformed with a vector comprising the nucleic acid of any of the above embodiments, wherein the nucleic acid comprises a nucleic acid fragment of a glucose oxidase mutant with an amino acid sequence such as SEQ ID NO:2 and a nucleic acid fragment of a glucose oxidase mutant with an amino acid sequence such as SEQ ID NO:3.

In one embodiment, the host cell is a cell that has cloned the nucleic acid fragment encoding the aforementioned glucose oxidase mutant.

In one embodiment, the host cell is a cell expressing the nucleic acid fragment encoding the aforementioned glucose oxidase mutant.

In one embodiment, the host cell comprises a recipient cell, with the nucleic acid encoding the glucose oxidase mutant or the vector located within the recipient cell.

In one embodiment, the recipient cells are selected from the group comprising *Escherichia coli, Agrobacterium, Saccharomyces cerevisiae, Pichia pastoris, Aspergillus niger*, animal cells, and plant cells. Further, the recipient cells can be *Escherichia coli* DH5α, *Escherichia coli* Top10, *Escherichia coli* Orgami(DE3), *Agrobacterium* AGL1, *Aspergillus niger*, *Pichia pastoris* GS115, or *Pichia pastoris* SMD1168. It should be noted that the recipient cells are not limited to the aforementioned recipient cells, and other common recipient cells can also serve as the recipient cells of the host cells.

The first embodiment of the present application further provides an application of the aforementioned nucleic acid, the aforementioned vector, and the aforementioned host cell in the preparation of glucose oxidase.

An embodiment of the present application further provides a method for preparing a glucose oxidase mutant, comprising the following steps: introducing at least one of the following mutations: G108K, G419K, G108D, G419D, into a glucose oxidase having an amino acid sequence such as SEQ ID NO:1.

Specifically, the steps for introducing at least one of the G108K, G419K, G108D, and G419D mutations into glucose oxidase comprise Steps S100 to S300.
Step S100: Preparation of a linear recombinant vector comprising the glucose oxidase gene;
Step S200: PCR amplifying the linear recombinant vector using primers as set forth in SEQ ID NOs: 10 to 13 and SEQ ID NOs: 14 to 17, respectively, to obtain a first mutant recombinant vector and a second mutant recombinant vector.
Step S300: Transforming the first mutant recombinant vector and the second mutant recombinant vector into host cells, respectively, culturing the host cells, and preparing the glucose oxidase mutant.

In one embodiment, step S100 comprises Steps S110-S120, wherein:
Step S110: PCR amplifying the glucose oxidase using primers as set forth in SEQ ID NOs: 6 to 7 to obtain a glucose oxidase target fragment.
Step S120: Mixing the glucose oxidase target fragment with a linearized ligation vector and reacting the mixture to ligate the glucose oxidase target fragment with the ligation vector, thereby obtaining the linear recombinant vector.

In some embodiments, each 20µL reaction system comprises 1 µL to 5µL of the glucose oxidase target fragment and 1µL -5µL of the linearized ligation vector.

Specifically, the nucleotide sequence set forth in SEQ ID NO:6 is: 5'-ATGCAGACTCTCCTTGTGAGC-3'; the nucleotide sequence set forth in SEQ ID NO:7 is: 5'- TCACTGCATGGAAGCATAATCTT -3'.

In some embodiments, step S120 includes Steps S121 to S122:
Step S121: Mixing the glucose oxidase target fragment with the linearized ligation vector and recombinase, respectively, and incubating at 37°C for at least 30 minutes to obtain a ligation product;
Step S122: Heat-shocking the ligation product into competent host cells and screening for monoclonal positive recombinants to obtain the linear recombinant vector.

In some embodiments, the recombinase is Exnase II.

In some embodiments, the heat-shock temperature is 40°C to 45°C, and the heat-shock time is 40 to 50 seconds.

In some embodiments, the heat-shock temperature is 42°C and the heat-shock time is 45 seconds.

In some embodiments, the host cell is *Escherichia coli* DH5α.

In some embodiments, Step S200 includes Steps S210 to S230:
Step S210: PCR amplifying the linear recombinant vector using primers as set forth in SEQ ID NOs: 10 to13 and SEQ ID NOs: 14 to 17 to obtain a first amplification product and a second amplification product, respectively. Step S220: Mixing the first and second amplification products with restriction endonucleases, respectively, and incubating at 37°C for at least 60 minutes to obtain a first digestion product and a second digestion product, respectively. Step S230: Heat-shocking the first and second digestion products into competent host cells, respectively, and screening for monoclonal positive recombinants to obtain the first mutant recombinant vector and the second mutant recombinant vector, respectively.

Specifically, the nucleotide sequence set forth in SEQ ID NO: 10 is: 5'-GTGAATAAAGGCACCTGGACTCGCCCCCACAA -3'; the nucleotide sequence set forth in SEQ ID NO:11 is: 5'-CAGGTGCCTTTATTCACTAGAGTAGAGCCACCGAGA -3'; the nucleotide sequence set forth in SEQ ID NO: 12 is: 5'- AGGACCCCAAACTTCACCACTTCGCCTACGACC -3'; the nucleotide sequence set forth in SEQ ID NO: 13 is: 5'-GTGAAGTTTGGGGTCCTTGTCGAGGATGTGAA -3'; the nucleotide sequence set forth in SEQ ID NO: 14 is: 5'- GTGAATGACGGCACCTGGACTCGCCCCCACAA -3'; the nucleotide sequence set forth in SEQ ID NO: 15 is: 5'-CAGGTGCCGTCATTCACTAGAGTAGAGCCACCGAGA -3'; the nucleotide sequence set forth in SEQ ID NO:16 is: 5'- AGGACCCCGACCTTCACCACTTCGCCTACGACC-3'; the nucleotide sequence set forth in SEQ ID NO: 17 is: 5'-GTGAAGGTCGGGGTCCTTGTCGAGGATGTGAA -3'.

In some embodiments, the PCR amplification reaction is programmed as follows: pre-denaturation at 95°C for 3 to 5 minutes; denaturation at 95°C for 10 to 30 seconds, annealing at 56°C to 60°C for 10 to 30 seconds, extension at 72°C for 1 to 5 minutes, for a total of 30 to 35 cycles; finally, extension at 72°C for 3 to 7 minutes, and storing the PCR amplification products at 4°C. It is understood that in other specific examples, the PCR program may be appropriately adjusted.

In one example, the PCR amplification program is set as: pre-denaturation at 95°C for 3 minutes; denaturation at 95°C for 15 seconds, annealing at 60°C for 15 seconds, extension at 72°C for 3 minutes, for a total of 30 cycles; finally, extension at 72°C for 5 minutes, and storing the PCR amplification products at 4°C.

In some embodiments, the restriction endonuclease is DPN1.

In some embodiments, the heat-shock temperature is 40°C to 45°C, and the heat-shock time is 40 to 50 seconds.

In some embodiments, the heat-shock temperature is 42°C and the heat-shock time is 45 seconds.

In some embodiments, the host cell is *Escherichia coli* DH5α.

In some embodiments, step S300 specifically involves transforming the first mutant recombinant vector and the second mutant recombinant vector into *Agrobacterium,* co-transforming with *Aspergillus niger,* and then fermenting the transformed *Aspergillus niger* to prepare the glucose oxidase mutant.

In some embodiments, the fermentation temperature is 30°C to 35°C.

In some embodiments, the fermentation time is 3 days to 8 days.

In some embodiments, the transformed host cells are fermented and cultured, and fermentation products are collected to prepare a primary product containing the glucose oxidase mutant; the primary product is then purified to prepare the purified glucose oxidase mutant.

In some embodiments, the present application provides an application of the aforementioned glucose oxidase mutant in the preparation of a biosensing element, which comprises a bioenzyme electrode or a biosensor.

The present application provides a bioenzyme electrode, which comprises a substrate electrode loaded with a modified material; the modified material comprises at least one of the aforementioned glucose oxidase mutants.

In some embodiments, the substrate electrode is selected from at least one of a glassy carbon electrode, a gold electrode, a graphite electrode, and a carbon paste electrode.

In some embodiments, the present application provides a method for preparing the aforementioned bioenzyme electrode, which comprises preparing the bioenzyme electrode by co - incubating the substrate electrode with the aforementioned glucose oxidase mutant.

In some embodiments, the method for preparing the substrate electrode comprises preparing the substrate electrode by coating a binder on the substrate electrode material.

In some embodiments, the binder comprises at least one of metal ions, proteins, or small molecule substances.

In some embodiments, the aforementioned metal ions include at least one of cobalt ions, calcium ions, zinc ions, nickel ions, copper ions, or trivalent iron ions.

In some embodiments, the aforementioned proteins include at least one of glutathione, Flag tag antibody, or calmodulin.

In some embodiments, the small molecule substances include at least one of amylose, biotin, chitin, or chlorinated alkanes.

In some embodiments, the present application provides a biosensor, which comprises an electrode of the aforementioned bioenzyme.

By site - directed mutagenesis, the immobilization of glucose oxidase has a uniform structural orientation with little loss of the activity of glucose oxidase, and then a suitable substrate electrode is selected to prepare a bioenzyme electrode, which is applied to the preparation of a biosensor that enables direct electron transfer between glucose oxidase and the surface of the substrate electrode, with a higher current level and a higher sensitivity level.

The following is detailed in combination with specific embodiments. In the following embodiments, unless otherwise specified, components other than unavoidable impurities are not included. The reagents and instruments used in the embodiments are, unless otherwise specified, customary choices in the art. For experimental methods where specific conditions are not specified in the examples, they are carried out under conventional conditions, such as those described in literature, books, or methods recommended by the manufacturer.

### Example 1

### 1. Preparation of Recombinant Expression Plasmid pMJB1-GOx

A glucose oxidase gene (GOx) fragment was obtained by PCR amplification using primer sequences 5'- ATGCAGACTCTCCTTGTGAGC-3' (SEQ ID NO:6) and 5'-TCACTGCATGGAAGCATAATCTT-3' (SEQ ID NO:7), with a glucose oxidase-producing strain as a template. The target band and non-specific bands were separated via DNA agarose gel electrophoresis. A solution containing the glucose oxidase gene was obtained after gel recovery.

Forward primers 5'-TATCCATACGATGTTCCTGATTATGC-3' (SEQ ID NO:8) and reverse primers 5'-ATACTGTTTAAATTAATCTATGCTAGCTTATTT-3' (SEQ ID NO:9) were designed. The vector pMJB 1 was linearized using a homologous recombination method and subsequently recombined with the glucose oxidase gene solution to obtain the pMJB 1-GOx expression plasmid.

The plasmid was linearized by polymerase chain reaction (PCR) using a DNA polymerase such as commercial PrimerSTAR Max Premix (2X). The preparation of the PCR reaction solution is shown in Table 1.

**Table 1**

| Reagents | Usage amount (µL) |
|---|---|
| PrimeSTAR Max Premix (2X) | 15 |
| Forward primer | 1 |
| Reverse primer | 1 |
| pMJB1 template | 1 |
| Sterilized water | 12 |

Place the prepared PCR reaction system into a PCR instrument and perform amplification according to the program set forth in Table 2 below.

**Table 2**

| Temperature (°C) | Time | Cyclic Number |
|---|---|---|
| 95 | 3 min | 0 |
| 95 | 15 s | 30 |
| 60 | 15 s | |
| 72 | 3 min | |
| 72 | 5 min | 0 |
| 4 | Hold | 0 |

After the PCR reaction was completed, the reaction solution was analyzed by DNA agarose gel electrophoresis, and the linearized pMJB1 vector was obtained after gel extraction.

### 2. Construction of the Recombinant Expression Plasmid pMJB 1 - Gox

The above - mentioned recovered linearized pMJB1 vector was ligated with the glucose oxidase gene fragment using homologous recombinase, and the reaction was set up as shown in Table 3. The prepared reaction solution was incubated at 37 °C for 30 minutes and then immediately transferred to 4 °C for cooling and storage.

**Table 3**

| Reagents | Usage amount (µL) |
|---|---|
| 5X CE II Buffer | 4 |
| Exnase II | 2 |
| The glucose oxidase gene | 4 |
| pMJB1 linearization template | 2 |
| Sterilized water | 8 |

Add the pMJB1 - Gox recombinant product to DH5α competent cells thawed on ice, mix well, incubate on ice for 30 min, transfer to a 42 °C water bath for heat - shock for 45 s, then cool on ice for 2 - 3 min, add 1 mL of -antibiotic-free LB liquid medium, shake at 37 °C for 1 hour, centrifuge at 2000 rpm, remove the supernatant, resuspend the bacterial pellet with the remaining medium, spread the bacterial suspension with a spreader onto an LB plate medium with ampicillin resistance, incubate overnight at 37°C, and send the plate for sequencing to obtain the successfully ligated expression plasmid of pMJB 1 - Gox.

3. Construction of the Recombinant Mutant Expression Plasmid pMJB 1-GOx-G108K/G419K

Using the pMJB1-GOx plasmid as a template, glycine residues at positions 108 and 419 were mutated to lysine. The point mutation primers are set forth in Table 4, respectively.

**Table 4**

| | Forward Primer (5 '-3') | Reverse Primer (5 '-3') |
|---|---|---|
| G108 K | GTGAATAAAGGCACCTGGACTCGCCCC CACAA(SEQ ID NO:10) | CAGGTGCCTTTATTCACTAGAGTAGAGCCA CCGAGA(SEQ ID NO:11) |
| G419 K | AGGACCCCAAACTTCACCACTTCGCCT ACGACC(SEQ ID NO:12) | GTGAAGTTTGGGGTCCTTGTCGAGGATGTG AA(SEQ ID NO:13) |

Point mutation was performed using PrimerStar Max Premix (2X) via PCR. The preparation of the PCR reaction solution is shown in Table 5.

**Table 5**

| Reagents | Usage Amount (µL) |
|---|---|
| PrimeSTAR Max Premix (2X) | 10 |
| Forward primer | 1 |
| Reverse primer | 1 |
| Gene template | 1 |
| Sterilized water | 7 |

Place the prepared PCR reaction system into a PCR instrument and perform amplification according to the program set forth in Table 6 below.

**Table 6**

| Temperature (°C) | Time | Cycles Number |
|---|---|---|
| 95 | 3 min | 0 |
| 95 | 15 s | 30 |
| 60 | 15 s | |
| 72 | 3 min | |
| 72 | 5 min | 0 |
| 4 | Hold | 0 |

After the PCR reaction is completed, add 1µL of DPN1 restriction endonuclease to the reaction mixture, incubate at 37°C for 1 hour, then cool the PCR product to 4°C. Add the mixture to DH5α competent cells thawed on ice, mix gently, incubate on ice for 30 minutes, transfer to a 42°C water bath for heat-shock for 45 seconds, and then cool on ice for 2 minutes to 3 minutes. Add 1mL of antibiotic-free LB liquid medium, incubate at 37°C with shaking for 1 hour, centrifuge at 2000rpm, discard the supernatant, resuspend the bacterial pellet in the remaining medium, spread the bacterial suspension with a spreader onto an LB plate medium containing ampicillin resistance, incubate overnight at 37°C, and submit the plate for sequencing. The successfully ligated expression plasmids of pMJB1-GOx-G108K/G419K were obtained, and glucose oxidase gene fragments of wild-type and mutant were obtained by DNA agarose gel electrophoresis.

### 4. Acquisition of Wild-Type Glucose Oxidase and Mutant Glucose Oxidase

The constructed expression plasmid was transformed into *Agrobacterium* AGL1 via the freeze-thaw method. Correctly identified *Agrobacterium* transformants were picked and cultured in liquid YEB medium for activation. A 50 µL aliquot of the activated *Agrobacterium* culture was mixed with 50 µL of *Aspergillus niger* mycelium with high glucoamylase activity and statically cultured in liquid potato medium, then evenly spread onto PDA plates containing 200 µmol/L acetosyringone (AS) lined with cellophane. After incubation at 28°C for 3 days, the cellophane was transferred onto PDA plates containing 250 mg/L hygromycin B and 400 mg/L cefotaxime sodium for 1 day. The cellophane was then removed, and the plates were further incubated at 32°C for 7 days to 10 days until visible *Aspergillus niger* growth was observed. *Aspergillus niger* was subsequently fermented, and the fermentation products were purified to obtain wild-type glucose oxidase (GOx) and mutant glucose oxidase (GOx-G108K/G419K). The uniform structural orientation of glucose oxidase was verified by Western blotting, confirming the expression of glucose oxidase with uniform structural orientation, as shown in FIG. 2.

### 5. Addition of Specific Tags

A 10*His polypeptide chain was synthesized in vitro and mixed with glucose oxidase in PBS buffer. Through dehydration condensation, the 10*His tag was conjugated to free amino groups of glucose oxidase using carbodiimide condensing agents such as dicyclohexylcarbodiimide (DCC) and acylation catalysts such as 4-N,N-dimethylaminopyridine (DMAP), forming 10*His-GOx wild-type glucose oxidase and 10*His-GOx-G108K/G419K mutant glucose oxidase.

### 6. Redox Potential Detection

Ferrocene methanol was used as an electron mediator in combination with the wild-type/mutant glucose oxidase obtained above. The specific steps were as follows: after polishing and ultrasonic cleaning, 5 µL of ferrocene methanol solution was applied to the surface of each glassy carbon electrode and allowed to dry naturally. Wild-type and mutant glucose oxidase solutions were then drop-cast onto the electrode surface, air-dried, and cured in a glutaraldehyde vapor environment for 30 minutes, followed by overnight drying in an oven at 45°C. After fixation, the redox potential of the modified electrodes was measured using an electrochemical workstation in a phosphate buffer system with an Ag/AgCl reference electrode and a platinum counter electrode, as shown in FIG. 3.

### 7. Testing Electrochemical Performance in Glucose Buffers at Different Concentrations

An electrode pattern was obtained by screen-printing conductive paste layer-by-layer on a substrate, followed by printing an insulating medium paste between layers and finalizing the base electrode through laser cutting and cleaning. The working electrode and counter electrode of the base electrode were composed of platinum-carbon paste, while the reference electrode layer was silver/silver chloride paste. The obtained 10*His-tagged glucose oxidase (wild-type/mutant) was dissolved in BSA solution. The matrix electrode-cobalt ion complex was immersed in a BSA solution containing 10*His-tagged glucose oxidase and glutaraldehyde for 10 minutes, then incubated at room temperature for 1 hour to obtain the glucose oxidase-coated sensing electrode. Electrochemical performance tests were conducted in buffers containing different concentrations of glucose.

The results are shown in FIGS. 4 to 5. Figure 4 depicts the relationship between the current response of the electrochemical sensor and glucose concentration, while FIG. 5 shows the linear correlation between the current and glucose concentration.

### Example 2

### 1. Preparation of the Recombinant Expression Plasmid pMJB1 - Gox

A glucose oxidase gene (GOx) fragment was obtained by PCR amplification using primer sequences 5'- ATGCAGACTCTCCTTGTGAGC-3' (SEQ ID NO:6) and 5'-TCACTGCATGGAAGCATAATCTT-3' (SEQ ID NO:7), with a glucose oxidase - producing strain as a template. The target band and non-specific bands were separated by DNA agarose gel electrophoresis. A solution containing the glucose oxidase gene was obtained after gel recovery.

Forward primers 5'-TATCCATACGATGTTCCTGATTATGC-3' (SEQ ID NO:8) and reverse primers 5'-ATACTGTTTAAATTAATCTATGCTAGCTTATTT-3' (SEQ ID NO:9) were designed. The vector pMJB 1 was linearized using the homologous recombination method and then recombined with the glucose oxidase gene solution to obtain the pMJB1 - Gox expression plasmid.

Plasmid linearization was carried out by polymerase chain reaction (PCR) using a DNA polymerase such as the commercial PrimerSTAR Max Premix (2X). The preparation of the PCR reaction solution is shown in Table 7.

**Table 7**

| Reagents | Usage Amount (µL) |
|---|---|
| PrimeSTAR Max Premix (2X) | 15 |
| Forward primer | 1 |
| Reverse primer | 1 |
| pMJB1 template | 1 |
| Sterilized water | 12 |

Place the prepared PCR reaction system into a PCR instrument and perform amplification according to the program shown in Table 8 below.

**Table 8**

| Temperature (°C) | Time | Cycles Number |
|---|---|---|
| 95 | 3 min | 0 |
| 95 | 15 s | 30 |
| 60 | 15 s | |
| 72 | 3 min | |
| 72 | 5 min | 0 |
| 4 | Hold | 0 |

After the PCR reaction is completed, the reaction solution is subjected to DNA agarose gel electrophoresis, and the linearized pMJB 1 vector is obtained after gel recovery.

### 2. Construction of the Recombinant Expression Plasmid pMJB 1 - Gox

The above - mentioned recovered linearized pMJB 1 vector was ligated with the glucose oxidase gene fragment using homologous recombinase, and the reaction was set up as shown in Table 9. The prepared reaction solution was incubated at 37 °C for 30 minutes and then immediately transferred to 4 °C for cooling and storage.

**Table 9**

| Reagents | Usage Amount (µL) |
|---|---|
| 5X CE II Buffer | 4 |
| Exnase II | 2 |
| The glucose oxidase gene | 4 |
| pMJB1 linearized template | 2 |
| Sterilized water | 8 |

The pMJB 1 - GOx recombinant product was added to DH5α competent cells thawed on ice, mixed well, incubated on ice for 30 min, transferred to a 42 °C water bath for heat - shock for 45 s, then placed on ice for cooling for 2 min to 3 min. One milliliter of -antibiotic-free LB liquid medium was added, and the mixture was incubated at 37 °C for 1 hour. After centrifugation at 2000 rpm, the supernatant was removed, and the bacterial pellet was resuspended in the remaining medium. The bacterial suspension was spread with a spreader onto an LB plate medium with ampicillin resistance, incubated overnight at 37 °C, and the plate was sent for sequencing to obtain the successfully ligated expression plasmid of pMJB 1 - GOx.

### 3. Construction of the Recombinant Mutant Expression Plasmid pMJB 1-GOx-G108D/G419D

Using the pMJB 1-GOx plasmid as a template, glycine residues at positions 108 and 419 were mutated to aspartic acid. The point mutation primers are set forth in Table 10, respectively.

**Table 10**

| | Forward Primer (5 '-3') | Reverse Primer (5 '-3') |
|---|---|---|
| G108 D | GTGAATGACGGCACCTGGACTCGCCCC CACAA(SEQ ID NO:14) | CAGGTGCCGTCATTCACTAGAGTAGAGCCA CCGAGA(SEQ ID NO:15) |
| G419 D | AGGACCCCGACCTTCACCACTTCGCCT ACGACC(SEQ ID NO:16) | GTGAAGGTCGGGGTCCTTGTCGAGGATGT GAA(SEQ ID NO:17) |

Point mutation was performed using PrimerStar Max Premix (2X) via PCR. The preparation of the PCR reaction solution is shown in Table 11.

**Table 11**

| Reagents | Usage Amount (µL) |
|---|---|
| PrimeSTAR Max Premix (2X) | 10 |
| Forward primer | 1 |
| Reverse primer | 1 |
| Plasmid template | 1 |
| Sterilized water | 7 |

Place the prepared PCR reaction system into a PCR instrument and perform amplification according to the program set forth in Table 12 below.

**Table 12**

| Temperature (°C) | Time | Cycles Number |
|---|---|---|
| 95 | 3min | 0 |
| 95 | 15s | 30 |
| 60 | 15s | |
| 72 | 3min | |
| 72 | 5min | 0 |
| 4 | Hold | 0 |

After the PCR reaction is completed, add 1µL of DPN1 restriction endonuclease to the reaction mixture and incubate at 37°C for 1 hour. The PCR product is then cooled to 4°C and added to DH5α competent cells thawed on ice. After gentle mixing, the mixture is incubated on ice for 30 minutes, transferred to a 42°C water bath for heat-shock for 45 seconds, and then cooled on ice for 2-3 minutes. Add 1mL of antibiotic-free LB liquid medium, incubate at 37°C with shaking for 1 hour, centrifuge at 2000rpm, discard the supernatant, resuspend the bacterial pellet in the remaining medium, spread the bacterial suspension with a spreader onto an LB plate medium containing ampicillin resistance, incubate overnight at 37°C, and submit the plate for sequencing. The successfully ligated expression plasmids of pMJB1-GOx-G108D/G419D were obtained, and glucose oxidase gene fragments of wild-type and mutant were obtained by DNA agarose gel electrophoresis.

### 4. Acquisition of Wild-Type Glucose Oxidase and Mutant Glucose Oxidase

The constructed expression plasmid was transformed into *Agrobacterium* AGL1 via the freeze-thaw method. Correctly identified *Agrobacterium* transformants were picked and cultured in liquid YEB medium for activation. A 50 µL aliquot of the activated *Agrobacterium* culture was mixed with 50 µL of *Aspergillus niger* mycelium with high glucoamylase activity and statically cultured in liquid potato medium, then evenly spread onto PDA plates containing 200 µmol/L acetosyringone (AS) lined with cellophane and cultured at 28°C for 3 days. The cellophane was transferred onto PDA plates containing 250 mg/L hygromycin B and 400 mg/L cefotaxime sodium and incubated for 1 day, after which the cellophane was removed, and the plates were further incubated at 32°C for 7 days to 10 days until visible *Aspergillus niger* growth was observed. *Aspergillus niger* was fermented and expressed, and fermentation products were purified to obtain wild-type glucose oxidase (GOx) and mutant glucose oxidase (GOx-G108D/G419D). The uniform structural orientation of glucose oxidase was verified by Western blotting, confirming the expression of glucose oxidase with uniform structural orientation, as shown in FIG. 6.

### 5. Addition of Specific Tags

A 10*His polypeptide chain was synthesized in vitro and mixed with glucose oxidase in PBS buffer. Through dehydration condensation, the 10*His tag was conjugated to free amino groups of glucose oxidase using carbodiimide condensing agents such as dicyclohexylcarbodiimide (DCC) and acylation catalysts such as 4-N,N-dimethylaminopyridine (DMAP), forming 10*His-GOx wild-type glucose oxidase and 10*His-GOx-G108D/G419D mutant glucose oxidase.

### 6. Redox Potential Detection

Ferrocene was used as an electron mediator in combination with the wild-type/mutant glucose oxidase obtained above. The specific steps were as follows: a ferrocene solution was applied to the surface of the substrate electrode and allowed to dry naturally, followed by drop-casting the glucose oxidase solution onto the electrode surface. The electrode was cured in a glutaraldehyde vapor environment, using an Ag/AgCl reference electrode and a platinum counter electrode. The redox potential of the modified electrode was measured with an electrochemical workstation, as shown in FIG. 7.

The above data indicate that the glucose oxidase mutant exhibits improved electron transfer capability, reflected in the bioelectrochemical sensor as higher current and sensitivity levels of the bioenzyme electrode coated with the glucose oxidase mutant at the same glucose concentration, demonstrating the mutant's superior electron transfer efficiency.

The technical features of the above embodiments may be combined in any manner. For brevity, not all possible combinations are described herein, but any combination of technical features without conflict shall be deemed within the scope of this disclosure.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present application. For example, the terms "one embodiment," "an embodiment," and "some embodiments" mean that a particular feature, structure or characteristic described in connection with the embodiment is included in at least one embodiment of the present application. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present application.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present application, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claim subject matter lie in less than all features of a single foregoing disclosed embodiment.

In some embodiments, the numbers expressing quantities or properties used to describe and claim certain embodiments of the application are to be understood as being modified in some instances by the term "about", "approximate", or "substantially". For example, "about", "approximate", or "substantially" may indicate a certain variation (e.g., ±1%, ±5%, ±10%, or ±20%) of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable. In some embodiments, a classification condition used in classification or determination is provided for illustration purposes and modified according to different situations. For example, a classification condition that "a value is greater than the threshold value" may further include or exclude a condition that "the probability value is equal to the threshold value."

## Claims

1. A glucose oxidase mutant, **characterized in that** the glucose oxidase mutant has at least one of the following mutations compared to wild-type glucose oxidase: G108K, G419K; or the glucose oxidase mutant has at least one of the following mutations compared to wild-type glucose oxidase: G108D, G419D;
the amino acid sequence of the wild-type glucose oxidase is set forth in SEQ ID NO: 1.

2. The glucose oxidase mutant according to claim 1, **characterized in that** the amino acid sequence of the glucose oxidase mutant is set forth in SEQ ID NO:2.

3. The glucose oxidase mutant according to claim 1, **characterized in that** the amino acid sequence of the glucose oxidase mutant is set forth in SEQ ID NO:3.

4. A nucleic acid encoding the glucose oxidase mutant according to any one of claims 1-3.

5. A vector comprising the nucleic acid according to claim 4.

6. A host cell comprising the vector according to claim 5.

7. The host cell according to claim 6, **characterized in that** the host cell is a recipient cell, preferably the recipient cell is selected from the group comprising: *Escherichia coli, Agrobacterium*, *Saccharomyces cerevisiae*, *Pichia pastoris, Aspergillus niger*, an animal cell, or a plant cell; more preferably, the recipient cell is selected from the group comprising: *Escherichia coli* DH5α, *Escherichia coli* Top10, *Escherichia coli* Origami(DE3), *Agrobacterium* AGL1, *Aspergillus niger*, *Pichia pastoris* GS115, or *Pichia pastoris* SMD1168.

8. A method for preparing a glucose oxidase mutant according to any one of claims 1-3, the method comprising introducing at least one of the following mutations: G108K, G419K into a glucose oxidase having an amino acid sequence as set forth in SEQ ID NO: 1; or introducing at least one of the following mutations: G108D, G419D into the glucose oxidase.

9. The method according to claim 8, **characterized in that** the method comprising steps S100 to S300:
step S100: preparing a linear recombinant vector, the linear recombinant vector comprising the glucose oxidase gene;
step S200: PCR amplifying the linear recombinant vector using primers as set forth in SEQ ID NOs: 10-13 and SEQ ID NOs: 14-17 to obtain a first mutant recombinant vector and a second mutant recombinant vector, respectively.
step S300: transforming the first mutant recombinant vector and the second mutant recombinant vector into host cells, culturing the host cells, and preparing the glucose oxidase mutant.

10. The method according to claim 8 or 9, **characterized in that** each 20µL reaction system comprises 1µL to 5µL of the glucose oxidase target fragment, and/or each 20µL reaction system comprises 1µL to 5µL of a linearized connecting carrier.

11. The method according to claim 9 or 10, **characterized in that** the PCR amplification reaction is programmed as: pre-denaturation at 95°C for 3 to 5 minutes; denaturation at 95°C for 10 to 30 seconds, annealing at 56°C to 60°C for 10 to 30 seconds, extension at 72°C for 1 to 5 minutes, for a total of 30-35 cycles; and finally, extension at 72°C for 3 to 7 minutes, and storing the PCR amplification products at 4°C.

12. Use of the glucose oxidase mutant according to any one of claims 1-3 in the preparation of a biosensing element, wherein the biosensing element comprises a biological enzyme electrode or a biosensor.

13. A biological enzyme electrode, comprising a base electrode loaded with a modified material for modifying the electrode;
the modified material comprises at least one of the glucose oxidase mutants according to any one of claims 1-3.

14. A method for preparing the biological enzyme electrode according to claim 13, the method comprising co-incubating a substrate electrode with the glucose oxidase mutant according to any one of claims 1-3.

15. A biosensor comprising the biological enzyme electrode according to claim 13.
